# EUROPÄISCHE PATENTSCHRIFT

(11) **EP 2 165 193 B1**
(45) Veröffentlichungstag und Bekanntmachung des Hinweises auf die Patenterteilung: **31.08.2016**
(21) Anmeldenummer: 08756847.3
(22) Anmeldetag: 02.07.2008
(51) Int. Cl.: C12M 1/34, C12Q 1/04

(54) **Vorrichtung zur Überwachung von Wasser auf mikrobielle Keime**
Apparatus for monitoring water for microbial germs
Dispositif de surveillance de la présence de germes microbiens dans l'eau

(30) Priorität: 09.07.2007 AT 10562007
(43) Veröffentlichungstag der Anmeldung: 24.03.2010
(73) Patentinhaber: microLAN B.V., 5145 PZ Waalwijk (NL)
(72) Erfinder: LENDENFELD, Thomas, A-3100 St. Pölten (AT)
(74) Vertreter: Patentanwaltskanzlei Matschnig & Forsthuber OG
(86) Internationale Anmeldenummer: PCT/AT2008/000238
(87) Internationale Veröffentlichungsnummer: WO 2009/006657

(56) Entgegenhaltungen:
- EP-A- 0 304 406
- DE-U1- 8 623 413
- GB-A- 2 365 122
- US-A- 4 613 434
- US-A1- 2007 003 997
- US-B1- 6 245 224
- US-B1- 6 818 185

## Beschreibung

Die Erfindung bezieht sich auf eine Vorrichtung zur fortlaufenden, periodischen Überwachung von Wasser auf mikrobielle Keime, mit einem Reaktor, zumindest einer Wasserzuleitung für das zu untersuchende Wasser in den Reaktor, zumindest einer Wasserableitung aus der Reaktorkammer, zumindest einer Reagenzienzuleitung in den Reaktor und zumindest einer Dosiereinrichtung für Reagenzien, zumindest einer Messeinrichtung für den Nachweis des Vorhandenseins von Mikroorganismen und/oder deren Stoffwechselprodukten, mit Pumpen/Ventilmitteln in den Zu/Ableitungen des Reaktors sowie mit einer Steuerung zur programmierten Steuerung zumindest der Pumpen/Ventilmittel.

Unter dem Begriff "Wasser' wird im Zusammenhang mit der Erfindung jede Art von Flüssigkeit verstanden, deren Hauptbestandteil Wasser ist, beispielsweise Trinkwasser, Mineralwasser, zur Trinkwasserherstellung verwendetes Wasser, Fluss- und Seewasser, technische Prozesswässer, z.B. Kühlwasser, Kreislaufwasser, biotechnologische Prozesslösungen, z.B. Fermentationslösungen, Abwasser und gereinigtes Abwasser etc. und Lebensmittel, deren Hauptbestandteil Wasser ist, z.B. Milch und Milchprodukte oder Produkte der Getränkeindustrie.

Bei derartigen Wässern ist es oft erwünscht bzw. auch auf Grund gesetzlicher Bestimmungen erforderlich, bereits geringe Belastungen durch Mikroorganismen detektieren zu können, wobei hier als repräsentativ der Nachweis von E. Coli genannt sei.

Man versucht seit längerer Zeit, Verfahren und Geräte zu schaffen, die einerseits den Nachweis bereits geringster Belastungen ermöglichen und die andererseits einen solchen Nachweis in möglichst kurzer Zeit gestatten, sodass eine praktisch kontinuierliche Wasseruntersuchung möglich ist.

Die genannte Problematik ist auch dem Dokument DE 86 23 413 U1 zu entnehmen. Dieses Dokument offenbart ein Messgerät zur Ermittlung coliformer Bakterien in Wasserproben, bei welchem durch die Bakterien erzeugter Wasserstoff nachgewiesen wird. Das Messgerät verwendet einen oder mehrere verschlossene Inkubationsbehälter, in welche das zu untersuchende Wasser, ein Nährmedium und zur Reinigung ein Desinfektionsmittel (Chlorwasser) eingebracht werden kann. Ausgangspunkt dieses bekannten Messgerätes ist die Ansicht, dass eine Vorkonzentration von Bakterien aus vielerlei Gründen nicht wünschenswert ist, insbesondere, da Filter verstopft und die Bakterienkonzentration am Filter zu hoch werden könnten und außerdem die Vorkonzentration auf der Filtermembran irreversibel sei. Dies führe dazu, dass die Verwendung einer Filtermembran eine einfache Automatisierung nicht ermöglichte.

Eine andere, praktisch kontinuierlich arbeitende Vorrichtung geht aus der JP 2003079397 hervor. Hier wird die zu untersuchende Probe mittels einer Pumpe über einen Zwischenbehälter, ein Dreiwegeventil und eine weitere Pumpe in eine Mischkammer gepumpt und mit Hilfe von Ventilen und einer Pumpe werden Reagenzien in die Mischkammer gepumpt. Von hier gelangt die Probe in einen eine Kapillare aufweisenden Inkubator. Nach dem Inkubator wird die Probe in eine weitere Mischkammer geführt, wo erneut über ein Ventil und eine Pumpe ein Reagens zugefügt wird. Nach Durchlaufen noch einer weiteren Kapillare gelangt die Probe in eine Messzelle, wo sie fluoreszenzoptisch gemessen wird. Die beschriebene Vorrichtung kann jedoch einerseits nicht die erforderliche Empfindlichkeit erreichen, um die Anforderungen an Trinkwasser zu erfüllen, andererseits ist der Verbrauch an Reagenzien durch die kontinuierliche Betriebsweise relativ hoch, wodurch erhebliche Kosten im laufenden Betrieb zu erwarten sind.

WO 2005/083109 A1 beschreibt ein Verfahren zum Messen von Kontaminationen in einem Medium, wobei ein bestimmtes Volumen des Mediums durch ein Filter geleitet wird, sodann die an der Zuflussseite liegende Seite des Filters mit einem Substrat in Kontakt gebracht wird, das in Wechselwirkung mit einem Kontaminant eine detektierbare Komponente erzeugt; diese Druckschrift offenbart keine Vorrichtung, die das Messverfahren ausführt. US 2007/0003997 A1 beschreibt ein Verfahren und eine Vorrichtung zur Messung von Bakterien in einer Flüssigkeitsprobe.

Falls geringe Konzentrationen von Keimen beispielsweise in Trinkwasser nachgewiesen werden sollen, werden daher nach wie vor Stichproben genommen, zu einem Labor gebracht, wo allfällig vorhandene Keime nach Inkubieren auf einem Nährmedium nachgewiesen werden.

Es besteht somit nach wie vor ein Bedarf an einer automatisierten Vorrichtung hoher Nachweisempfindlichkeit und die Erfindung hat sich die Aufgabe gesetzt, eine solche Vorrichtung zu schaffen, wobei von dem Vorhandensein eines Filters ausgegangen wird, da nach Auffassung der Erfinder dadurch die gewünschte Nachweisempfindlichkeit relativ einfach erzielt und bewährte Reagenzien anwendbar sind. Ziel des Einsatzes eines Filters ist jedoch nicht die Elimination von Partikeln oder Bakterien, um eine gereinigte Lösung zu erhalten, wie es beispielsweise in der Biotechnologie häufig angewandt wird, sondern ein Aufkonzentrieren der Bakterien, um bei einer nachfolgenden Messung die notwendige Sensitivität zu erreichen.

Diese Aufgabe wird mit einer Vorrichtung der eingangs genannten Art gelöst, bei welcher erfindungsgemäß der Reaktor eine Reaktorkammer und eine von dieser durch ein Filter getrennte Filtratkammer aufweist, wobei die zumindest eine Wasserzuleitung sowie die zumindest eine Reagenzienzuleitung in die Reaktorkammer münden und die zumindest eine Wasserableitung aus der Filtratkammer führt, und die Steuerung dazu eingerichtet ist, mittels der Pumpen/Ventilmittel eine vorgebbare Wassermenge in die Reaktorkammer und durch das Filter zu führen sowie eine vorgebbare Reagenzienmenge in die Reaktorkammer einzuleiten, die Steuerung dazu eingerichtet ist, mittels der Pumpen/Ventilmittel den Strömungsfluss des Wassers durch den Reaktor umzukehren, und die Filtratkammer über der Reaktorkammer liegt.

Dank der Erfindung ist eine Einrichtung geschaffen, die ein automatisiertes Messen über längere Zeiträume ermöglicht und dank ihrer Ausgestaltung vielseitig einsetzbar ist, sodass beispielsweise keine Einschränkung auf bestimmte Keime, Nachweismethoden oder Qualität des zu untersuchenden Wassers gegeben ist Durch die Umkehr des Strömungsflusses des Wassers durch den Reaktor kann einerseits im Rückfluss gemessen werden, wodurch die in manchen Fällen durch das Passieren des Filters auftretende Verdünnung und damit verbunden eine Signalreduktion nicht auftritt, und andererseits kann dadurch die Reinigung der Apparatur erleichtert werden.

Bei einer besonders günstig realisierbaren Ausführung ist vorgesehen, dass die Messeinrichtung zur Messung stromab der Filtratkammer eingerichtet ist.

Zur Erhöhung der Empfindlichkeit kann es zweckmäßig sein, wenn vor der Messeinrichtung eine Konzentrationseinheit angeordnet ist. Dabei hat es sich als besonders wirksam erwiesen, wenn die Konzentrationseinheit nach dem Prinzip der chromatographischen Trennung arbeitet.

Die Flexibilität der Vorrichtung kann dadurch gesteigert werden, dass die Messeinrichtung zur Messung in der Reaktorkammer und/oder zur Messung in der Filtratkammer eingerichtet ist.

Es ist weiters in vielen Fällen empfehlenswert, wenn zumindest der Reaktor eine Heizung aufweist, um reproduzierbare Bedingungen für die biologischen/chemischen Abläufe zu schaffen.

Für einen geregelten Ablauf der in der Vorrichtung laufenden Prozesse ist es verteilhaft, wenn eine Druckmesseinrichtung für den Druck in der Wasserzuleitung zu der Reaktorkammer vorgesehen ist.

Falls ein Rührmittel in der Reaktorkammer vorgesehen ist, wird eine gleichmäßige Durchmischung der reagierenden Stoffe sichergestellt.

Unter bestimmten Brut- und/oder Messbedingungen kann es weiters sinnvoll sein, wenn die Steuerung dazu eingerichtet ist, mittels der Pumpen/Ventilmittel den Strömungsfluss des Wassers durch den Reaktor im Kreis zu führen.

Da der erhaltene Messwert zweckmäßigerweise auf ein bestimmtes Volumen zu beziehen ist, ist es zweckmäßig, wenn die Steuerung dazu eingerichtet ist, das über die Pumpe geförderte Messvolumen zu erfassen und/oder vorzugeben.

Im Sinne eines weitgehend automatisierbaren Arbeitsablaufes ist es empfehlenswert, wenn der Filter in dem Reaktor auswechselbar ist. Dabei zeichnet sich eine zweckmäßige Weiterbildung dadurch aus, dass eine Filterwechselplatte mit zumindest zwei Filtereinsätzen vorgesehen ist, welche, gegen das Reaktorgehäuse abgedichtet, so verschiebbar ist, dass jeweils ein Filtereinsatz als wirksamer Filter Reaktor- und Filtratkammer trennt bzw. dass der Filterwechselplatte ein von der Steuerung angesteuerter Antrieb zugeordnet ist.

Da aus unterschiedlichen Gründen in dem Reaktor bzw. dessen Kreislauf Luft- oder Gasblasen entstehen und zu einer Beeinträchtigung des Messablaufes führen können, ist es ratsam, diese aus dem Reaktor zu entfernen.

Dabei hat es sich in der Praxis als besonders zweckmäßig erwiesen, wenn der Filter im Wesentlichen eben ausgebildet und gegen die Horizontale so geneigt ist, dass Gasblasen in der Reaktorkammer über die Filteroberfläche nach oben streichen können. Dabei ist es besonders günstig, wenn die Filtratkammer Ableitungsöffnungen zum Ableiten des Wassers in eine erste und eine zweite Wasserableitung aufweist, wobei die Ableitungsöffnung der ersten Wasserableitung am höchsten Punkt der Filtratkammer und die Ableitungsöffnung der zweiten Wasserableitung, welche das Wasser der Messeinrichtung zuführt, in einer im Wesentlichen normal zur Filterebene verlaufenden Symmetrieachse des Reaktors angeordnet ist. Beim Ableiten des Wassers am höchsten Punkt der Filtratkammer können vorhandene Gas- und Luftblasen, die über den gegen die Horizontale geneigten Filter nach oben streichen und den Filter passieren, effektiv über die Ableitungsöffnung der ersten Wasserableitung entweichen. Beim Ableiten des in die Messeinrichtung zuzuführenden Wassers aus der Filtrationskammer über die in der im Wesentlichen normal zur Filterebene verlaufenden Symmetrieachse des Reaktors gelegene Ableitungsöffnung der zweiten Wasserableitung können Durchmischungseffekte und eine damit verbundene Signalreduktion klein gehalten werden.

Ferner können zum Entfernen von Luft- oder Gasblasen aus dem Reaktor zumindest für die Reaktorkammer eine Entlüftungsleitung und ein Entlüftungsventil vorgesehen sein. Falls eine Filterwechselplatte vorhanden ist, bietet sich die Lösung an, dass die Filterwechselplatte eine Entlüftungsausnehmung aufweist, die in einer definierten Verschiebestellung der Platte eine Verbindung zwischen Reaktor- und/oder Filtratkammer und einer nach außen führen den Entlüftungsleitung freigibt.

Zur Gewährleistung eines automatisierten, quasikontinuierlichen Betriebes trägt eine Weiterbildung bei, bei welcher sie eine Reinigungseinheit aufweist und die Steuerung durch Steuerung der Pumpen/Ventilmittel dazu eingerichtet ist, in zumindest einem Reinigungszyklus Reinigungsfluida durch die Leitungen und Einheiten der Einrichtung zu führen.

Die Erfindung samt weiterer Vorteile ist im Folgenden an Hand beispielsweiser Ausführungsformen in der Zeichnung veranschaulicht, in welcher zeigen
Fig.1 in schematischer Blockdarstellung eine erste Ausführungsform der Erfindung,
Fig. 2 in einer vergrößerten Darstellung ein Detail einer Ausführung nach der Erfindung,
Fig. 3 bis 6 verschiedene Flusswege während des Arbeitsablaufes in Darstellungen entsprechend Fig. 1, wobei jedoch für die Erläuterung unwesentliche Teile weggelassen sind, und
Fig. 7 in einer Darstellung ähnlich Fig. 1 eine Ausführungsform der Erfindung mit erweiterten Funktionalitäten.

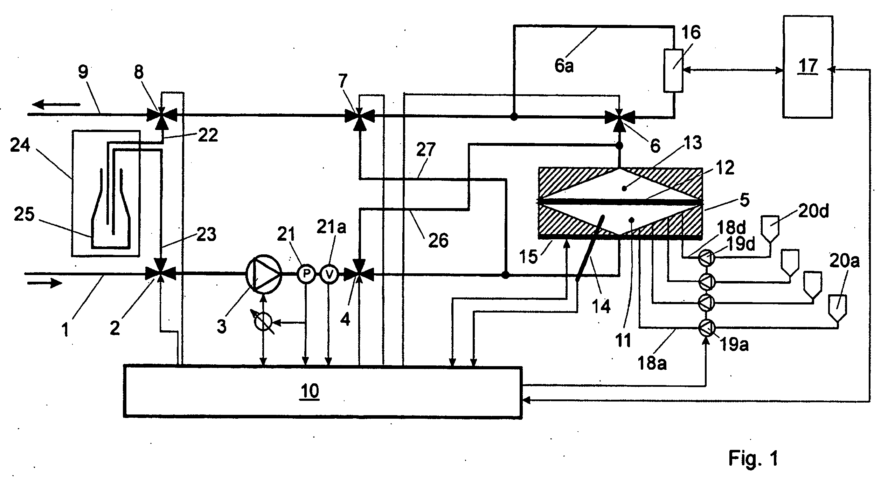

Gemäß Fig. 1 wird das zu untersuchende Wasser - zur Definition von "Wasser" siehe weiter oben - über eine Wasserzuleitung 1 ein erstes 3-Wege Ventil 2, eine Pumpe 3 und ein zweites 3-Wege Ventil 4 zu einem Reaktor 5 geführt und von dort über ein drittes 3-Wege Ventil 6, ein viertes 3-Wege Ventil 7 und ein fünftes 3-Wege Ventil 8 über eine Wasserableitung 9 wieder abgeleitet.

Sämtliche 3-Wege Ventile sind von einer zentralen Steuerung 10 angesteuert bzw. durch diese betätigbar, wobei es klar sein sollte, dass der soeben beschriebene Kreislauf nur eine von mehreren Möglichkeiten bei bestimmten Stellungen der 3-Wege Ventile darstellt. Die Steuerung 10 ist sinnvollerweise auch zur zentralen Datenerfassung und zur Datenausgabe eingerichtet.

Der Reaktor 5 besitzt eine Reaktorkammer 11 und eine, hier darüberliegende, durch ein Filter 12 getrennte Filtratkammer 13. Die Temperatur innerhalb des Reaktors kann mit Hilfe eines Temperatursensors 14 erfasst und eine entsprechende Information an die Steuerung 10 weitergeleitet werden. Andererseits kann der Reaktor 5 mit Hilfe einer Heizung 15 beheizt werden, die über die Steuerung, zweckmäßigerweise ausgehend von der Information des Temperatursensors 14 geregelt wird.

In von dem dritten 3-Wege Ventil 6 ausgehenden Messzweig 6a ist eine Messeinrichtung 16 angeordnet, die mit einer Peripherieeinheit 17 für diese Messeinrichtung in Verbindung steht, wobei andererseits letztere mit der Steuerung 10 kommunizieren kann.

Über Reagenzienzuleitungen 18a,18b, 18c und 18d und diesen zugeordnete, von der Steuerung 10 ansteuerbare Dosierventile 19a, 19b, 19c und 19d können Reagenzien aus Vorratsbehältern 20a, 20b, 20c und 20d in die Reaktorkammer 11 eingebracht werden.

Zwischen der Pumpe 3 und dem zweiten 3-Wege Ventil 4 ist eine Druckmesseinrichtung 21 angeordnet, welche den Wert des aktuellen Druckes an die Steuerung 10 weiterleitet, so dass diese, beispielsweise über die Förderleistung der Pumpe 3, den Druck auf einem vorgebbaren Wert halten kann.

Von dem ersten 3-Wege Ventil 2 bzw. dem fünften 3-Wege Ventil 8 führt je ein Abzweig 22 bzw. 23 zu einer Reinigungseinrichtung 24, die im gezeigten Ausführungsbeispiel zumindest ein Gefäß 25 für ein Reinigungsfluidum enthält, wobei hier keine Einschränkung auf flüssige Reinigungsmittel erfolgt und auch andere Möglichkeiten, wie z.B. Generation von Ozon, zur Reinigung herangezogen werden können

Eine Querleitung 26 verbindet den Ausgang der Filterkammer 13 mit dem zweiten 3-Wege Ventil 4, eine Querleitung 27 den Eingang der Reaktorkammer 11 mit dem vierten 3-Wege Ventil 7.

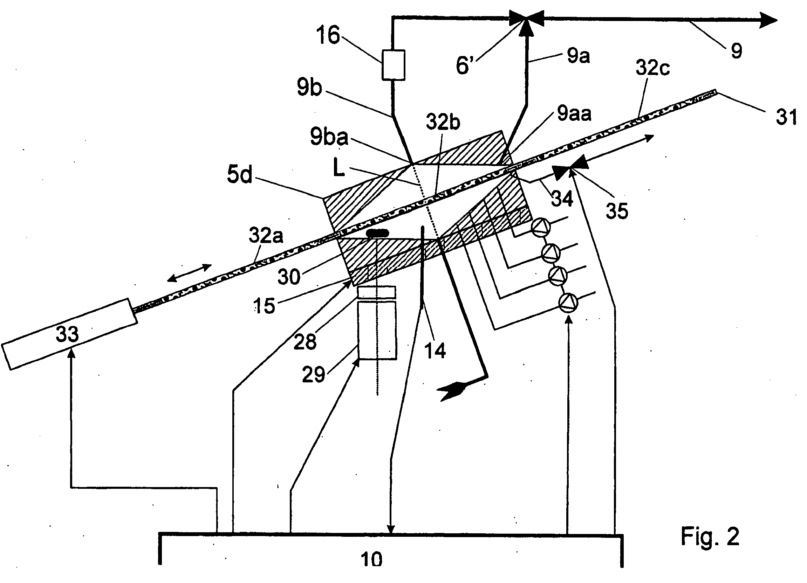

Bevor auf die Funktion bzw. die verschiedenen Betriebsarten einer Vorrichtung nach der Erfindung eingegangen wird, seien noch weitere Details anhand der Fig. 2 erläutert, in welcher gleiche Bezugszeichen für gleiche Teile wie in Fig.1 verwendet werden und bereits in Fig. 1 erläuterte Teile weggelassen sind.

Die Fig. 2 zeigt einen Reaktor 5d, welcher in Bezug auf die Horizontale schräg geneigt ist. Durch die Neigung des Reaktors 5d ist auch die Filterplatte 31 geneigt.

Um in der Reaktorkammer 11 eine bestimmte Flüssigkeitsbewegung aufrechterhalten zu können, ist hier ein Magnetrührer 28 vorgesehen, der einen Motor 29 und im Inneren der Kammer 11 einen Rührmagnet 30 aufweist. Der Motor 29 ist wiederum von der zentralen Steuerung 10 angesteuert. Selbstverständlich können andere Technologien eingesetzt werden, um eine Bewegung in der Reaktorkammer 11 aufrecht zu erhalten, sofern diese gewünscht ist Durch die Neigung des Reaktors 5d ergibt sich für den Rührmagneten 30 eine im Wesentlichen horizontale Fläche in der Reaktorkammer 11. Bei geeigneter Anordnung wird der Rührmagnet 30 aufgrund der Geometrie der Reaktorkammer 11 durch die Schwerkraft in der tiefsten Position gehalten.

Um ein verbrauchtes oder beschädigtes Filter automatisch wechseln zu können besitzt die Vorrichtung eine Filterwechselplatte 31 mit drei Filtereinsätzen 32a, 32b und 32c. Diese Filtereinsätze können beispielsweise handelsübliche Keramikfilter sein. Ein von der Steuerung 10 gesteuerter Antrieb 33 kann nun die Filterwechselplatte 31 verschieben, wobei diese gegen das Reaktorgehäuse abgedichtet ist, sodass jeweils ein anderer Filtereinsatz als wirksamer Filter die Reaktor- von der Filterkammer trennt In der gezeigten Stellung ist der Filtereinsatz 32b wirksam.

Aus unterschiedlichen Ursachen und an unterschiedlichen Stellen der Leitungsführung können Luft- oder Gasblasen entstehen, die von dem Filter aufgehalten und sich vor allem in der Reaktorkammer 11 störend auswirken können. Durch die Schrägneigung des Reaktors 5d und der Filterplatte 31 können etwaig vorhandene Luft- oder Gasblasen in der Reaktorkammer 11 entlang der Filteroberfläche nach oben streichen, durch den Filter 12 in die Filtratkammer 13 gepresst und abgeleitet werden. Bei der gezeigten Ausführungsform führen zwei Wasserableitungen 9a und 9b aus der Filtratkammer 13 des Reaktors 5d. Die Ableitungsöffnung 9aa der ersten Wasserableitung 9a liegt im Wesentlichen an der höchsten Stelle der Filtratkammer 13 und dient zum Ableiten des Wassers aus dem Reaktor 5d während des Filtrationsvorganges. Durch diese Anordnung können vorhandene Luftblasen, die in der Reaktorkammer entlang der Filteroberfläche nach oben streichen und den Filter 12 passieren, über die erste Wasserableitung 9a entweichen. Die Ableitungsöffnung 9ba der zweiten Wasserableitung 9b, über welche das Wasser der Messeinrichtung 16 zugeführt wird, ist in der Symmetrieachse L des Reaktors 5d angeordnet. Die Symmetrieachse L des Reaktors 5d verläuft dabei im Wesentlichen normal zur Filteroberfläche. Diese Anordnung hat den Vorteil, dass beim Messvorgang, bei dem die zu messende Lösung von der Reaktorkammer 11 durch den Filter 12 in die Filtratkammer 13 und von dort über die zweite Wasserableitung 9b in die Messeinrichtung 16 geführt wird, keine Diskriminierung der Reaktionslösung - aufgrund unterschiedlicher Wege von der Filteroberfläche zur Ableitungsöffnung 9ba der zweiten Wasserableitung 9b - stattfindet und daher Durchmischungseffekte und eine damit verbundene Signalreduktion klein gehalten werden. Die Wasserableitungen 9a und 9b münden über ein 3-Wege Ventil 6' in eine Wasserableitung 9.

Zusätzlich können Luft oder Gasblasen über eine Entlüftung des Reaktors 5d entfernt werden. Für letzteres können daher für die Reaktorkammer 11 eine Entlüftungsleitung 34 und ein Entlüftungsventil 35 vorgesehen sein, damit bei Bedarf oder zu bestimmten Zeiten des Verfahrensablaufes ein Entlüften durchgeführt werden kann. Es ist auch hier sinnvoll, wenn der üblicherweise eben ausgebildete Filter gegen die Horizontale so geneigt ist, dass Gasblasen in der Reaktorkammer 11 über die Filteroberfläche nach oben streichen und so zu dem Einlass der genannten Entlüftungsleitung 34 gelangen können.

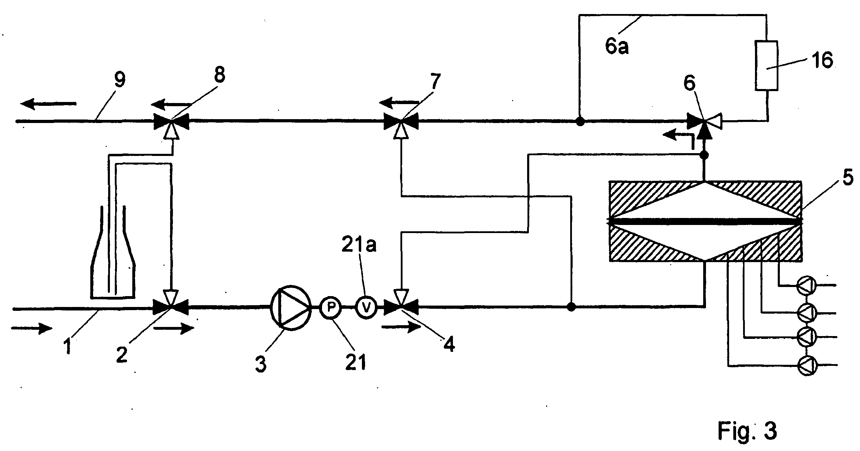

Im Weiteren wird unter Bezugnahme auf die Fig. 3 bis 6 der Betrieb und die Funktion einer Vorrichtung nach der Erfindung erklärt.

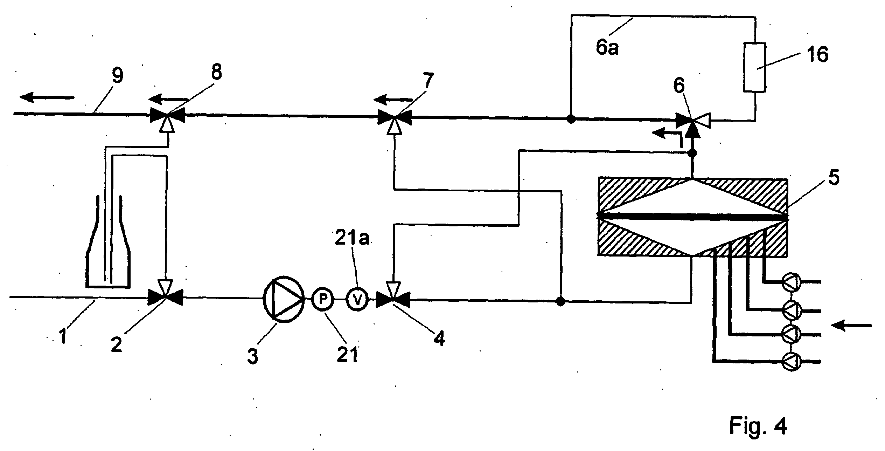

Fig. 3 zeigt die Ventilstellungen und den zugehörige Strömungsverlauf im Betriebsmodus "Filtration". Die Probe wird dabei über die Wasserzuleitung 1 mittels der Pumpe 3 laufend angesaugt und durch die Reaktorkammer 11, den Filter 12 und die Filtratkammer 13 bis zu der Ableitung 9 geführt. Um einen Bezug der später erhaltenen Messwertes auf ein Volumen zu ermöglichen, z.B. die Anzahl der Keime je 100 ml, wird das filtrierte Volumen an Wasserprobe gemessen, bzw. dosiert zugeführt. Hierzu können beispielsweise die Umdrehungen der Pumpe 3 erfasst werden oder es kann ein Strömungsmessgerät 21a zu Erfassung des durch die Pumpe 3 geförderten Volumens eingesetzt werden. Dieses Strömungsmessgerät 21a liefert eine entsprechende Information an die Steuerung 10 und diese kann nach Erreichen eines bestimmten geförderten Volumens die Pumpe 3 abschalten oder Ventile schließen. Mikrobielle Keime werden an dem Filter 12 zurückgehalten. An den Betriebsmodus "Filtration" schließt der Betriebsmodus "Dosierung und Inkubation" gemäß Fig. 4 an, in dessen Verlauf zunächst über die Reagenzienzuleitungen 18a, 18b, 18c und 18d und diesen zugeordnete, von der Steuerung 10 ansteuerbare Dosierventile 19a, 19b, 19c und 19d Reagenzien aus den Vorratsbehältern 20a, 20b, 20c und 20d in die Reaktorkammer 11 eingebracht werden. Das dabei verdrängte Volumen verlässt über den Filter den Reaktor, sodass das Volumen in der Reaktorkammer konstant bleibt. Das Zudosieren der Reagenzien kann wahlweise zu unterschiedlichen Zeiten der Inkubation erfolgen. Die über den Temperatursensor 14 und die Steuerung 10 geregelte Heizung 15 sowie der Magnetrührer 28 sorgen für optimale Reakrionsbedingungen

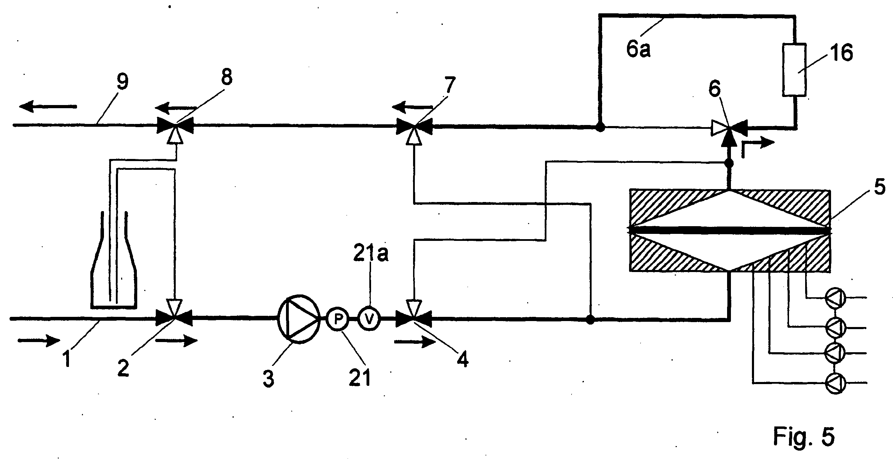

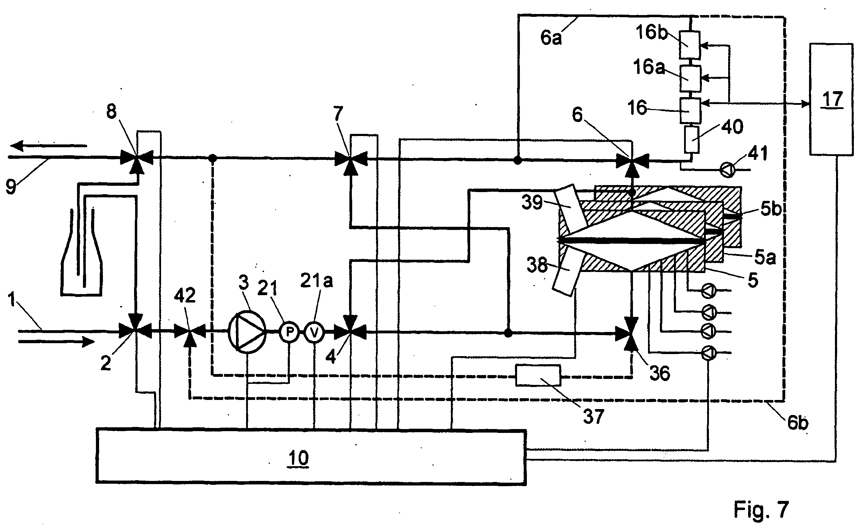

Der darauffolgende Messschritt erfolgt im Betriebsmodus "Messung" nach Fig. 5. Die Pumpe 3 wird erneut eingeschaltet und über die Zuleitung 1 strömendes Wasser drückt die in der Reaktorkammer 11 befindliche Messlösung durch den Filter 12 über die Filtratkammer 13 und von hier über das dritte 3-Wege Ventil 6 in eine Messzelle der Messeinrichtung 16. Der über die Peripherieeinheit 17 und/oder die Steuerung 10 ausgebbare Messwert beschreibt die Konzentration der Analyten in der Reaktorkammer zum Ende der Inkubation. Varianten der Messmöglichkeiten und Messorte werden weiter unten in Zusammenhang mit Fig. 7 beschrieben.

Als Beispiel für ein analytisches Verfahren sei hier der Nachweis von Escherichia coli (E.coli) angeführt. E. coli ist ein Indikator für fäkale Verunreinigung und wird weltweit routinemäßig zur Überwachung u.a. von Trinkwasser eingesetzt.

Ein definiertes Volumen an Probe wird mittels der Pumpe über den Filter in der Reaktorkammer abfiltriert In dieses Volumen der Reaktorkammer mit den angereicherten Bakterien werden Puffer- und Substratlösung zudosiert. Die Pufferlösung stellt für die enzymatische Reaktion ideale Bedingungen her, die Substratlösung enthält ein Substrat, welches selektiv durch ein bestimmtes Enzym von E.coli umgesetzt werden kann. Das gängige Substrat im Zusammenhang mit E.coli ist 4-Methylumbelliferyl-ß-D-Glucuronid, welches von dem für E.coli typischen Enzym ß-D-Glucuronidase gespalten wird und so das hochfluoreszierende 4-Methylumbelliferon freisetzt, welches fluoreszenzoptisch gemessen wird.

Durch die hohe Sensitivität der erfindungsgemäßen Vorrichtung, welche durch Kombination von Anreicherung und sensitiver Messung erreicht wird, kann auf eine Anzucht der Bakterien verzichtet werden. Daher können auch die Bedingungen im Zuge der Inkubation speziell auf jene der ß-Glucuronidase abgestimmt werden.

Nach entsprechender Inkubation, welche je nach zu erwartender Zahl an E.coli im Berich von 1-6 Stunden liegen kann, wird das Volumen der Reaktorkammer in der Messzelle 16 vermessen. Das erhaltene Signal ist ein Maß für die Anzahl der E.coli in der Reaktorkammer; mit dem bekannten, filtrierten Probenvolumen kann der Gehalt an Keimen pro Volumseinheit berechnet werden.

Alternativ kann ein Signalverlauf über die Zeit der Inkubation aufgenommen werden, wenn die Messzelle, bzw. Messeinrichtung in der Reaktorkammer eingesetzt wird.

Der Nachweis von E.coli mittels Nachweis selektiver enzymatischer Leistungen ist jedoch nur eine von mehreren Anwendungsmöglichkeiten der gegenständlichen Erfindung. Mit den weiter unten beschriebenen Modifikationen und Kombinationen können z.B. auch i.) Keime im Allgemeinen angefärbt und gemessen werden, wobei ii.) eine generelle Zellfärbung (z.B. Lebendfärbung) oder iii.) eine selektive Zellfärbung z.B. mit Antikörpern vorgenommen werden kann, oder auch iv.) Verfahren unter Zuhilfenahme der selektiven Anzucht mit anschließender Messung.

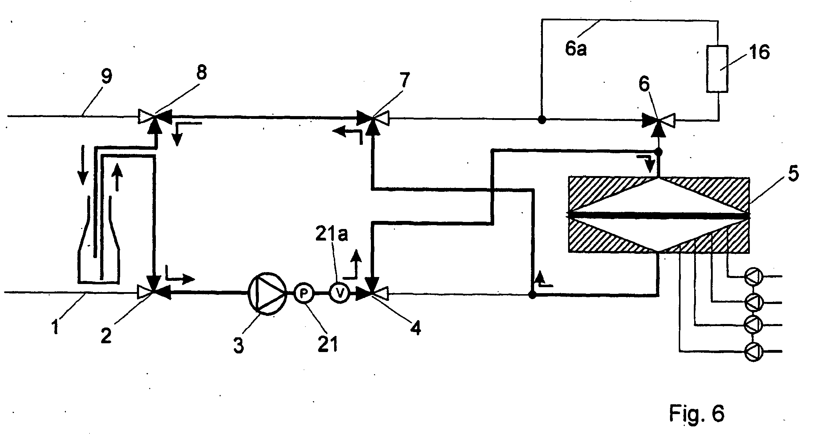

In dem Betriebsmodus "Rückspülung und Reinigung", siehe Fig. 6, können mehrere Schritte ablaufen, wobei eine Mehrfachdurchführung und/oder Wiederholung von Einzelschritten möglich ist

Dabei wird von der durch die Steuerung 10 gesteuerten Reinigungseinrichtung 24 aus dem Gefäß 25 für das Reinigungsfluidum, z.B. Chlorwasser, dieses Reinigungsmittel, gegebenen falls auch mehrere Reinigungsmittel, entnommen. Es erfolgt ein Durchspülen des Probenweges wie in dem Betriebsmodus "Filtration". Des Weiteren kann der Reaktor auch nur mit Wasser durchspült werden und es kann auch ein Rückspülen des Reaktors mit einem Reinigungsmittel erfolgen. Auch kann der Messzweig 6a in die Reinigung und/ oder Rückspülung einbezogen werden. Der gesamte Reinigungsablauf kann automatisch ablaufen und wird den jeweils vorliegenden Bedingungen, wie Art der zu detektierenden Miroorganismen, Reagenzien und Reinigungsmittel angepasst, so dass nach Ende des Reinigungsvorganges die Vorrichtung für die Messung einer neuen Probe zur Verfügung steht

Die in Fig. 7 gezeigte Ausführungsform besitzt gegenüber jener nach Fig. 1 einige Erweiterungen, z.B. einen zusätzlichen zweiten Reaktor 5a und dritten Reaktor 5b; manche, bereits in Fig.1 und 2 gezeigte Teile, wie Heizung und Rührwerk sind weggelassen, um die Darstellung zu vereinfachen.

Ein sechstes 3-Wege Ventil 36 vor dem Einlass in die Reaktorkammer 11 und eine zusätzliche/alternativeMesseinrichtung 37 im Rückfluss.

Je eine weitere Messeinrichtung 38 und 39 können in der bzw. für die Reaktorkammer 11 und die Filtratkammer 13 angeordnet bzw. vorgesehen sein, wobei die Messeinrichtung 38 für die Reaktorkammer 11 bevorzugt ist In dem Messzweig 6a können, der Messeinrichtung 16 nachgeschaltet, weitere Messeinrichtungen 16a und 16b angeordnet sein. Alternativ können diese Messeinrichtungen auch parallel geschaltet sein.

Ein siebentes 3-Wege Ventil 42 zwischen dem ersten 3-Wege Ventil 2 und der Pumpe 3 und der Messzweig 6b dienen der Kreislaufführung der Messlösung während der Inkubation. Vor den Messeinrichtungen 16, 16a, 16b ist eine Konzentrationseinheit 40 eingefügt, um die Empfindlichkeit der Messung zu steigern. Diese kann auf Chromatographiebasis beruhen, wobei über eine gesteuerte Pumpe 41 ein Eluent zum Herauslösen der angereicherten Analyten zugeführt werden kann.

## Patentansprüche

1. Vorrichtung zur fortlaufenden, periodischen Überwachung von Wasser auf mikrobielle Keime, mit
einem Reaktor (5),
zumindest einer Wasserzuleitung (1) für das zu untersuchende Wasser in den Reaktor, zumindest einer Wasserableitung (9,9a, 9b) aus der Reaktorkammer,
zumindest einer Reagenzienzuleitung (18a..d) in den Reaktor und zumindest einer DosierEinrichtung (19a..d) für Reagenzien
zumindest einer Messeinrichtung (16) für den Nachweis des Vorhandenseins von Mikroorganismen und/ oder deren Stoffwechselprodukten
mit Pumpen/Ventilmitteln (3, 4, 6, 7, 36,42) in den Zu/Ableitungen des Reaktors
sowie mit einer Steuerung (10) zur programmierten Steuerung zumindest der Pumpen/Ventilmittel,
**dadurch gekennzeichnet, dass**
der Reaktor (5) eine Reaktorkammer (11) und eine von dieser durch ein Filter (12) getrennte Filtratkammer (13) aufweist, wobei die zumindest eine Wasserzuleitung (1) sowie die zumindest eine Reagenzienzuleitung (18a...d) in die Reaktorkammer münden und die zumindest eine Wasserableitung (9, 9a, 9b) aus der Filtratkammer führt,
die Steuerung (10) dazu eingerichtet ist, mittels der Pumpen/Ventilmittel (3, 4, 6, 7) eine vorgebbare Wassermenge in die Reaktorkammer und durch das Filter zu führen sowie eine vorgebbare Reagenzienmenge in die Reaktorkammer einzuleiten,
die Steuerung (10) dazu eingerichtet ist, mittels der Pumpen/Ventilmittel (3, 4, 6, 7) den Strömungsfluss des Wassers durch den Reaktor (5) umzukehren, und
die Filtratkammer (13) über der Reaktorkammer (11) liegt.

2. Vorrichtung nach Anspruch 1, **dadurch gekennzeichnet, dass** die Messeinrichtung (16) zur Messung stromab der Filtratkammer (13) eingerichtet ist.

3. Vorrichtung nach Anspruch 1, **dadurch gekennzeichnet, dass** die Messeinrichtung (38) zur Messung in der Reaktorkammer (11) eingerichtet ist.

4. Vorrichtung nach Anspruch 1, **dadurch gekennzeichnet, dass** die Messeinrichtung (39) zur Messung in der Filtratkammer (13) eingerichtet ist

5. Vorrichtung nach Anspruch 1, **dadurch gekennzeichnet, dass** die Steuerung (10) dazu eingerichtet ist, mittels der Pumpen/Ventilmittel (3, 4, 6, 42) den Strömungsfluss des Wassers durch den Reaktor (5) im Kreis zu führen.

6. Vorrichtung nach Anspruch 1, **dadurch gekennzeichnet, dass** die Steuerung (10) dazu eingerichtet ist, dass über die Pumpe (3) geförderte Messvolumen zu erfassen und/oder vorzugeben.

7. Vorrichtung nach Anspruch 1, **dadurch gekennzeichnet, dass** der Filter (12) in dem Reaktor (5) auswechselbar ist.

8. Vorrichtung nach Anspruch 7, **dadurch gekennzeichnet, dass** eine Filterwechselplatte (31) mit zumindest zwei Filtereinsätzen (32a, b, c) vorgesehen ist, welche, gegen das Reaktorgehäuse abgedichtet, so verschiebbar ist, dass jeweils ein Filtereinsatz als wirksamer Filter (12) Reaktor- und Filtratkammer (11,13) trennt.

9. Vorrichtung nach Anspruch 8, **dadurch gekennzeichnet dass** der Filterwechselplatte (31) ein von der Steuerung (10) angesteuerter Antrieb (33) zugeordnet ist

10. Vorrichtung nach Anspruch 1, **dadurch gekennzeichnet, dass** der Filter (12) im Wesentlichen eben ausgebildet und gegen die Horizontale so geneigt ist, dass Gasblasen in der Reaktorkammer (11) über die Filteroberfläche nach oben streichen können,

11. Vorrichtung nach Anspruch 10, **dadurch gekennzeichnet, dass** die Filtratkammer 13 Ableitungsöffnungen (9aa,9ba) zum Ableiten des Wassers in eine erste und eine zweite Wasserableitung (9a,9b) aufweist, wobei die Ableitungsöffnung (9aa) der ersten Wasserableitung (9a) am höchsten Punkt der Filtratkammer (13) und die Ableitungsöffnung (9ba) der zweiten Wasserableitung (9b), welche das Wasser der Messeinrichtung (16) zuführt, in einer im Wesentlichen normal zur Filterebene verlaufenden Symmetrieachse (L) des Reaktors (5d) angeordnet ist.

12. Vorrichtung nach Anspruch 1, **dadurch gekennzeichnet, dass** zumindest für die Reaktorkammer (11) eine Entlüftungsleitung (34) und ein Entlüftungsventil (35) vorgesehen sind.

13. Vorrichtung nach Anspruch 8 und 12, **dadurch gekennzeichnet, dass** die Filterwechselplatte (31) eine Entlüftungsausnehmung aufweist, die in einer definierten Verschiebestellung der Platte eine Verbindung zwischen Reaktor- und/oder Filtratkammer (11, 13) und der nach außen führenden Entlüftungsleitung freigibt.

14. Vorrichtung nach Anspruch 1, **dadurch gekennzeichnet, dass** sie eine Reinigungseinrichtung (24, 25) aufweist und die Steuerung (10) durch Steuerung der Pumpen/Ventilmittel (2, 3, 4, 6, 7, 8) dazu eingerichtet ist, in zumindest einem Reinigungszyklus Reinigungsfluida durch die Leitungen und Einheiten der Einrichtung zu führen.

## Claims

1. A device for the continuous, periodic monitoring of water for microbial germs, having
a reactor (5),
at least one water supply line (1) for the water to be studied into the reactor,
at least one water drain line (9, 9a, 9b) out of the reactor chamber,
at least one reagent supply line (18 a...d) into the reactor and at least one dosing device (19 a...d) for reagents,
at least one measuring device (16) for the detection of the presence of microorganisms and/or the metabolic products thereof,
having pump/valve means (3, 4, 6, 7, 36, 42) in the supply/drain lines of the reactor, and having a controller (10) for the programmed control of at least the pump/valve means,
**characterized in that**
the reactor (5) has a reactor chamber (11) and a filtrate chamber (13) separated therefrom by a filter (12), the at least one water supply line (1) and the at least one reagent supply line (18a...d) opening into the reactor chamber and the at least one water drain line (9, 9a, 9b) leading out of the filtrate chamber,
the controller (10) is configured to conduct, by means of the pump/valve means (3, 4, 6, 7), a pre-definable water quantity into the reactor chamber and through the filter, as well as introducing a pre-definable reagent quantity into the reactor chamber,
the controller (10) is configured for the purpose of reversing the flow of the water through the reactor (5) by means of the pump/valve means (3, 4, 6, 7), and
the filtrate chamber (13) is positioned above the reactor chamber (11).

2. The device according to Claim 1, **characterized in that** the measuring device (16) is configured for the measurement downstream from the filtrate chamber (13).

3. The device according to Claim 1, **characterized in that** the measuring device (38) is configured for the measurement in the reactor chamber (11).

4. The device according to Claim 1, **characterized in that** the measuring device (39) is configured for the measurement in the filtrate chamber (13).

5. The device according to Claim 1, **characterized in that** the controller (10) is configured for the purpose of guiding the flow of the water through the reactor (5) in the circuit by means of the pump/valve means (3, 4, 6, 42).

6. The device according to Claim 1, **characterized in that** the controller (10) is configured for the purpose of detecting and/or predefining the measured volume conveyed via the pump (3).

7. The device according to Claim 1, **characterized in that** the filter (12) in the reactor (5) is replaceable.

8. The device according to Claim 7, **characterized in that** a filter replacement plate (31) having at least two filter inserts (32a, b, c) is provided which, sealed against the reactor housing, is displaceable so that one filter insert at a time separates reactor and filtrate chambers (11, 13) as an active filter (12).

9. The device according to Claim 8, **characterized in that** a drive (33) activated by the controller (10) is assigned to the filter replacement plate (31).

10. The device according to Claim 1, **characterized in that** the filter (12) is designed as essentially flat and is inclined to the horizontal so that gas bubbles in the reactor chamber (11) can sweep upward over the filter surface.

11. The device according to Claim 10, **characterized in that** the filtrate chamber (13) has drain line openings (9aa, 9ba) for draining the water into a first and a second water drain line (9a, 9b), the drain line opening (9aa) of the first water drain line (9a) being situated at the highest point of the filtrate chamber (13) and the drain line opening (9ba) of the second water drain line (9b), which supplies the water to the measuring device (16), being situated in an axis of symmetry (L) of the reactor (5d) running essentially perpendicular to the filter plane.

12. The device according to Claim 1, **characterized in that** a deaeration line (34) and a deaeration valve (35) are provided at least for the reactor chamber (11).

13. The device according to Claims 8 and 12, **characterized in that** the filter replacement plate (31) has a deaeration recess, which, in a defined displacement position of the plate, releases a connection between reactor chamber and/or filtrate chamber (11, 13) and the deaeration line leading to the outside.

14. The device according to Claim 1, **characterized in that** it has a cleaning device (24, 25) and the controller (10) is configured, by controlling the pump/valve means (2, 3, 4, 6, 7, 8), to conduct cleaning fluids through the lines and units of the device in at least one cleaning cycle.

## Revendications

1. Dispositif pour surveiller périodiquement, en continu, de l'eau sur des germes microbiens, comprenant :
un réacteur (5) ;
au moins une conduite d'alimentation en eau (1) pour l'eau devant être analysée dans le réacteur ;
au moins une conduite de décharge d'eau (9, 9a, 9b) sortant de la chambre de réacteur ;
au moins une conduite d'alimentation en réactifs (18a...d) dans le réacteur et au moins un dispositif de dosage (19a...d) pour les réactifs ;
au moins un dispositif de mesure (16) pour détecter la présence de microorganismes et/ou des produits de métabolisme de ceux-ci ;
comprenant des moyens de pompe/de soupape (3, 4, 6, 7, 36, 42) dans les conduites d'alimentation/décharge du réacteur ;
ainsi que comprenant un contrôleur (10) pour le contrôle programmé d'au moins les moyens de pompe/de soupape, **caractérisé par le fait que**
le réacteur (5) présente une chambre de réacteur (11) et une chambre de filtrat (13) qui est séparée de cette dernière par un filtre (12), la au moins une conduite d'alimentation en eau (1) ainsi que la au moins une conduite d'alimentation en réactifs (18a...d) débouchant dans la chambre de réacteur et la au moins une conduite de décharge d'eau (9, 9a, 9b) conduisant hors de la chambre de filtrat,
le contrôleur (10) est agencé de façon à, à l'aide des moyens de pompe/de soupape (3, 4, 6, 7), conduire une quantité d'eau pouvant être prédéfinie dans la chambre de réacteur et à travers le filtre ainsi qu'introduire une quantité de réactif pouvant être prédéfinie dans la chambre de réacteur ;
le contrôleur (10) est agencé de façon à, à l'aide des moyens de pompe/de soupape (3, 4, 6, 7), inverser le courant d'écoulement de l'eau à travers le réacteur (5) ; et
la chambre de filtrat (13) se trouve au-dessus de la chambre de réacteur (11).

2. Dispositif selon la revendication 1, **caractérisé par le fait que** le dispositif de mesure (16) est agencé pour la mesure en aval de la chambre de filtrat (13).

3. Dispositif selon la revendication 1, **caractérisé par le fait que** le dispositif de mesure (38) est agencé pour la mesure dans la chambre de réacteur (11).

4. Dispositif selon la revendication 1, **caractérisé par le fait que** le dispositif de mesure (39) est agencé pour la mesure dans la chambre de filtrat (13).

5. Dispositif selon la revendication 1, **caractérisé par le fait que** le contrôleur (10) est agencé de façon à, à l'aide des moyens de pompe/de soupape (3, 4, 6, 42), guider l'écoulement de courant de l'eau à travers le réacteur (5) en cercle.

6. Dispositif selon la revendication 1, **caractérisé par le fait que** le contrôleur (10) est disposé pour détecter et/ou prédéfinir des volumes de mesure transportés via la pompe (3).

7. Dispositif selon la revendication 1, **caractérisé par le fait que** le filtre (12) est remplaçable dans le réacteur (5).

8. Dispositif selon la revendication 7, **caractérisé par le fait qu'**une plaque de changement de filtre (31) comportant au moins deux cartouches filtrantes (32a, b, c) est prévue, laquelle, rendue étanche vis-à-vis de l'enveloppe de réacteur, est déplaçable de telle sorte qu'une cartouche filtrante respective en tant que filtre actif (12) sépare les chambres de réacteur et de filtrat (11, 13).

9. Dispositif selon la revendication 8, **caractérisé par le fait qu'**à la plaque de changement de filtre (31) est attribué un organe d'entraînement (33) commandé par le contrôleur (10).

10. Dispositif selon la revendication 1, **caractérisé par** le filtre (12) est réalisé sensiblement plat et est incliné par rapport à l'horizontale de telle sorte que des bulles de gaz dans la chambre de réacteur (11) peuvent être dirigées vers le haut sur la surface de filtre.

11. Dispositif selon la revendication 10, **caractérisé par le fait que** la chambre de filtrat (13) présente des ouvertures de conduite de décharge (9aa, 9ba) pour décharger l'eau dans une première et une deuxième conduite de décharge d'eau (9a, 9b), l'ouverture de conduite de décharge (9aa) de la première conduite de décharge d'eau (9a) étant disposée au point le plus haut de la chambre de filtrat (13) et l'ouverture de conduite de décharge (9ba) de la deuxième conduite de décharge d'eau (9b), laquelle adresse l'eau au dispositif de mesure (16), étant disposée dans un axe de symétrie (L) du réacteur (5d) qui s'étend sensiblement perpendiculairement au plan du filtre.

12. Dispositif selon la revendication 1, **caractérisé par le fait qu'**au moins pour la chambre de réacteur (11), sont prévues une conduite de ventilation (34) et une soupape de ventilation (35).

13. Dispositif selon l'une des revendications 8 et 12, **caractérisé par le fait que** la plaque de changement de filtre (31) présente une cavité de ventilation, qui libère une connexion entre la chambre de réacteur et/ou de filtrat (11, 13) et la conduite de ventilation conduisant vers l'extérieur dans une position de déplacement définie de la plaque.

14. Dispositif selon la revendication 1, **caractérisé par le fait qu'**il présente un dispositif de nettoyage (24, 25) et que le contrôleur (10) est agencé par la commande des moyens de pompe/de soupape (2, 3, 4, 6, 7, 8) de façon à guider dans au moins un cycle de nettoyage des fluides de nettoyage à travers les conduites et unités du dispositif.
